# EUROPÄISCHE PATENTSCHRIFT

(11) **EP 0 647 607 B1**
(45) Veröffentlichungstag und Bekanntmachung des Hinweises auf die Patenterteilung: **27.08.1997**
(21) Anmeldenummer: 94115414.8
(22) Anmeldetag: 30.09.1994
(51) Int. Cl.: C07C 17/367, C07C 21/185

(54) **Verfahren zur Herstellung von fluorierten Monomeren**
Process for the preparation of fluorinated monomers
Procédé de préparation de monomères fluorés

(30) Priorität: 06.10.1993 DE 4334015
(43) Veröffentlichungstag der Anmeldung: 12.04.1995
(73) Patentinhaber: Dyneon GmbH, 84504 Burgkirchen (DE)
(72) Erfinder: Schöttle, Thomas, Dr., D-84547 Emmerting (DE); Hintzer, Klaus, Dr., D-84556 Kastl (DE); Staudt, Hans Josef, Dr., D-84533 Haiming (DE); Weber, Herbert, D-84508 Burgkirchen (DE)

(56) Entgegenhaltungen:
- FR-A- 2 124 212
- GB-A- 1 496 347
- SYNTHESIS., Nr.6, 1982, STUTTGART DE Seite 454 R.J. HUNADI ET AL. 'Tetrafluoroethylene : A Convenient Laboratory Preparation.'

## Beschreibung

Die Rückgewinnung von fluorierten Monomeren durch Pyrolyse von Fluorpolymeren ist bekannt. So bezieht sich beispielsweise die US-A 3 832 411 auf ein Verfahren zur Herstellung von Tetrafluorethylen durch Pyrolyse von Polytetrafluorethylen, indem man die Pyrolyse durch Erhitzen mit Wasserdampf bei einer Temperatur zwischen etwa 405 und 760 °C und ohne Anwendung von Unterdruck durchführt, wobei das Molverhältnis des Wasserdampfs zu den Pyrolyseprodukten mindestens 1 : 1 beträgt. Bei diesem Verfahren wird das Polytetrafluorethylen auf eine poröse Fläche aufgebracht, beispielsweise auf ein Drahtsieb, und auch mit einem Siebmaterial abgedeckt oder umhüllt. Hierdurch soll verhindert werden, daß Teile des festen Rohmaterials durch den Dampf und die gasförmigen Zersetzungsprodukte nach oben geblasen werden. Das geschmolzene Polytetrafluorethylen fließt durch die poröse Unterlage und bildet hierbei Stalaktiten, wobei der vorhandene heiße Dampf das Polymerisat zersetzen muß, bevor Tropfen der Schmelze zum Boden des Behälters gelangen können. Die Durchführung und Steuerung dieses Verfahrens ist deshalb sehr aufwendig und praktisch nur absatzweise möglich.

Es wurde nun gefunden, daß dieses Verfahren gut beherrschbar wird, wenn man das zu pyrolysierende Fluorpolymerisat kontinuierlich in einen beheizten Wirbelschichtreaktor einträgt, der als Wirbelgut inertes, körniges Material enthält. Als Wirbelgas dient Wasserdampf.

Aus der Dissertation von Jürgen Merkel, Universität Hamburg, Fachbereich Chemie, 1982, ist die Pyrolyse von Polytetrafluorethylen in der Wirbelschicht bekannt. Bei diesem Verfahren wird das Polymere bei einer Temperatur von 720 bis 790 °C, speziell bei 760 und 790 °C, in ein Sandwirbelbett eingetragen. Im Unterschied zum erfindungsgemäßen Verfahren wird ein Teil der gasförmigen Spaltprodukte als Wirbelmedium im Kreislauf zum Reaktor zurückgeführt. Die Ausbeute an Tetrafluorethylen liegt hierbei unter 5 Gew.-%, die an Hexafluorpropen bei 22 Gew.-% und die von Perfluorcyclobutan (das als Dimeres des Tetrafluorethylens hier zu den gewünschten "Monomeren" gerechnet wird) und Perfluorbuten-1 zusammen bei 32 bis 37 Gew.-%. Bei niedrigeren Temperaturen (etwa 650 °C) werden etwa 38 Gew.-% wachsartige Produkte gebildet, die zu Wandanbackungen, Verklebungen und Verstopfungen führen. Weiterhin werden etwa 2 Gew.-% flüssige Produkte gebildet. Dieses Verfahren hat deshalb keine Bedeutung erlangt.

Im Gegensatz zu diesem bekannten Verfahren erhält man bei der erfindungsgemäßen Wirbelschichtmethode die gewünschten Monomeren in hohen Ausbeuten und praktisch ohne störende höhermolekulare Pyrolyseprodukte. Bevorzugte Ausgestaltungen der Erfindung werden im folgenden näher erläutert.

Als Ausgangsmaterialien eignen sich fluorierte Homo- und Copolymere in Form von reinen Polymeren, Mischungen verschiedener Polymerer und auch Mischungen von Polymeren mit Füllstoffen wie Kohlenstoff, Glas oder Metallen. Geeignet sind auch Pasten und Dispersionen solcher polymerer Stoffe. Bevorzugt wird das Homopolymerisat von Tetrafluorethylen, auch in Verbindung mit Füllstoffen wie Kohlenstoff in Form von Graphit oder Kohle, Glas oder Metallen wie Bronze. Generell sind alle Füllstoffe geeignet, die bei der Pyrolyse keine störenden Nebenprodukte bilden oder die die Pyrolyse selbst beeinträchtigen.

Das Wirbelbett wird durch den Eintrag von Wasserdampf als Wirbelmedium aufrechterhalten. Der Einsatz von Inertgasen als Wirbelmedium ist im allgemeinen nicht vorteilhaft, da diese die Abtrennung der gebildeten Monomeren erschweren.

Als Wirbelgut werden mineralische Stoffe wie Sand, Glas, Keramik, Metalloxide wie Aluminiumoxid und ähnliche Inertstoffe eingesetzt.

Die Pyrolysetemperatur liegt im allgemeinen zwischen 500 und 900 °C. Die zur Pyrolyse benötigte Wärme kann sowohl über den erhitzten Dampf als auch durch eine zusätzliche direkte oder indirekte Beheizung, beispielsweise der Reaktorwandung, zugeführt werden. Die bevorzugte Pyrolysetemperatur liegt im Bereich von 600 bis 750 °C, insbesondere im Bereich von 625 bis 675 °C.

Nach der Pyrolyse wird das Gemisch aus Wasserdampf und Pyrolyseprodukten in einem Zyklon von mitgerissenen Feststoffteilchen befreit und durch Einsprühen von Wasser schnell abgekühlt, wobei der Wasserdampf auskondensiert. Die Pyrolyseprodukte werden anschließend, beispielsweise mit konzentrierter Schwefelsäure, getrocknet und fraktioniert destilliert.

Das erfindungsgemäße Verfahren führt in hoher Ausbeute zu brauchbaren fluorierten Monomeren. Im Falle des Polytetrafluorethylens werden in nahezu quantitativer Ausbeute Tetrafluorethylen, Hexafluorpropen und Perfluorcyclobutan erhalten. Flüssige oder feste Pyrolysenebenprodukte werden nur in äußerst geringem Ausmaß gebildet.

In den folgenden Beispielen wird die Erfindung näher erläutert.

### Beispiele

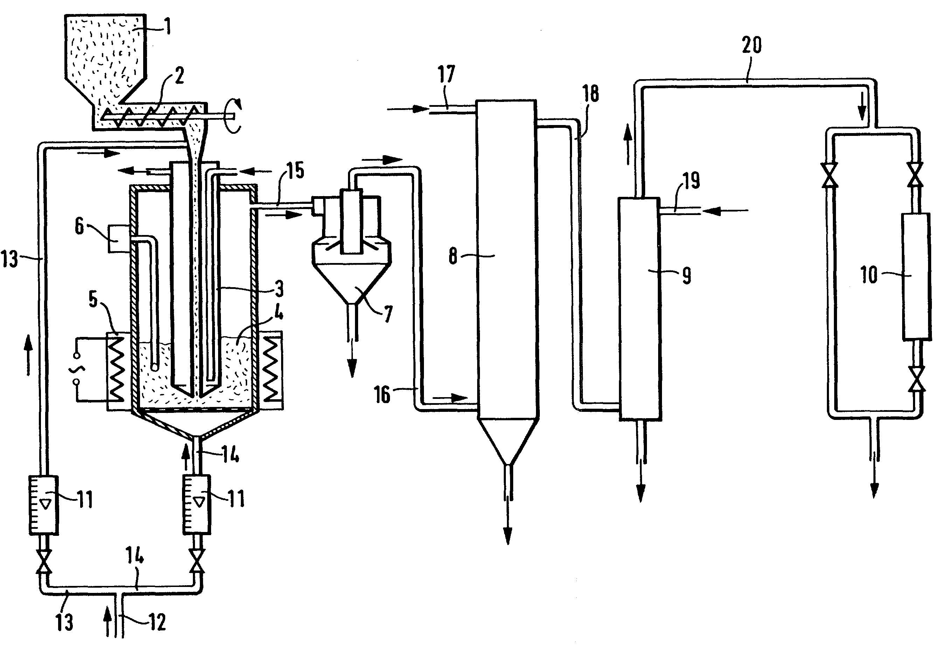

Die in den folgenden Beispielen eingesetzte Apparatur ist in der Figur schematisch dargestellt. In dieser bedeuten (1) einen Vorratsbunker, (2) eine Dosierschnecke, (3) ein kühlbares Tauchrohr, (4) die Wirbelschicht, (5) eine (elektrische) Heizung, (6) einen Temperaturfühler (Thermoelement), (7) einen Zyklon, (8) einen Kühler (Quenchkühler), (9) einen Wäscher, (10) einen Behälter mit Probenahmevorrichtung und (11) Gasmeßgeräte (Rotameter). (12) ist die Leitung vom Dampferzeuger, wobei der Dampf über die Leitungen (13) und (14) in die Wirbelschicht geleitet wird. Die Pyrolysegase gelangen über die Leitung (15) in den Zyklon (7) und von dort über die Leitung (16) in den Kühler (8), der über die Leitung (17) mit Wasser beschickt wird. Vom Kühler (8) gelangen dann die Pyrolysegase über die Leitung (18) in den Wäscher (9), der über die Leitung (19) mit Schwefelsäure beschickt wird. Die gewaschenen Pyrolysegase gelangen dann über die Leitung (20) in den Behälter mit Probenahmevorrichtung (10).

### Versuchsdurchführung:

Der Vorratsbunker (1) enthält gemahlenes Polytetrafluorethylen (PTFE) mit einem mittleren Partikeldurchmesser von etwa 0,5 mm. Dieses wird kontinuierlich mit Hilfe der Dosierschnecke (2) durch das gekühlte Fallrohr (3) mit Wasserdampf aus der Leitung (13) in die beheizte Sandwirbelschicht (4) geleitet. Diese Wirbelschicht besteht aus 140 g Seesand mit einer Partikelgröße von 0,3 bis 0,4 mm. Die Pyrolysetemperatur (Temperatur der Sandwirbelschicht) ist nachstehend in den Beispielen angegeben. Die Wirbelschicht ist bei einem Durchmesser von 7 cm 5 cm hoch. Von unten kommt durch die Leitung (14) zusätzlicher Wasserdampf (40 % der Gesamtmenge) durch den Anströmboden, der die Wirbelschicht (4) nach unten begrenzt. Die Wasserdampftemperatur beträgt 500 °C, die Eintragsgeschwindigkeit durch die Leitung (12) 5 g/min. Die Verteilung der Wasserdampfmenge, also 60 Vol.-% über die Leitung (13) und 40 Vol.-% über die Leitung (14), wird über Ventile und die Rotameter (11) gesteuert. Der Druck beträgt 1 bar absolut.

Die gasförmigen Pyrolyseprodukte verlassen mit dem Wasserdampf die Wirbelschicht (4) über die Leitung (15) und werden im Zyklon (7) von mitgerissenen Feststoffpartikeln befreit. Die Gase gelangen dann über die Leitung (16) in den Quenchkühler (8), in dem die Reaktionsgase durch Einspritzen von Wasser, eingespeist über die Leitung (17), gekühlt werden, wobei der Wasserdampf auskondensiert. Die Pyrolysegase gelangen dann über die Leitung (18) in den Wäscher (9), der über die Leitung (19) mit Schwefelsäure beschickt wird. Das durch die Schwefelsäure getrocknete Gasgemisch gelangt dann über die Leitung (20) in den Behälter (10) mit Probenahmevorrichtung.

Die variablen Versuchsparameter zeigt die Tabelle 1, die Versuchsergebnisse die Tabelle 2.

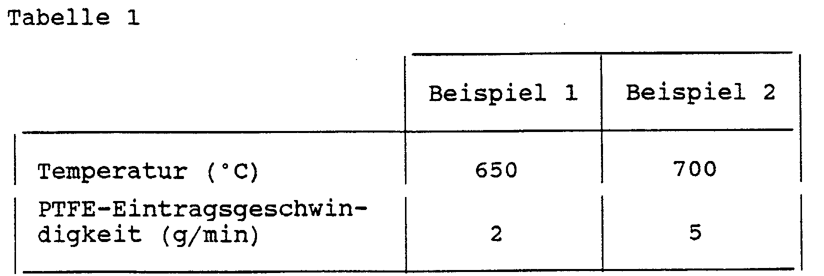

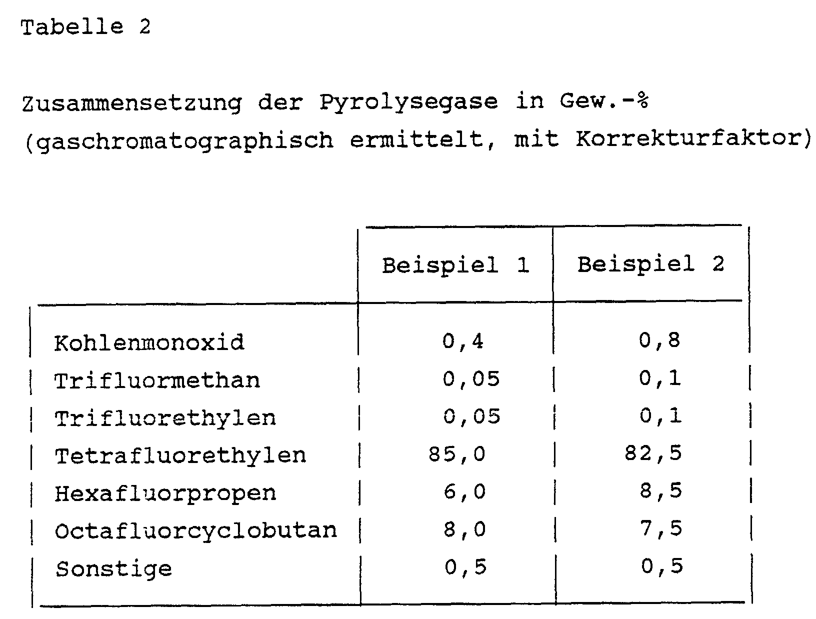

## Patentansprüche

1. Verfahren zur Herstellung von fluorierten Monomeren durch Pyrolyse von fluorierten Polymeren in Gegenwart von Wasserdampf, dadurch gekennzeichnet, daß man die Umsetzung in einem Wirbelschichtreaktor durchführt, der als Wirbelgut inertes, körniges Material enthält, und als Wirbelgas Wasserdampf zugeführt wird.

2. Verfahren nach Anspruch 1, dadurch gekennzeichnet, daß man ein Polymer des Tetrafluorethylens einsetzt.

3. Verfahren nach Anspruch 1 oder 2, dadurch gekennzeichnet, daß die Pyrolyse bei einer Temperatur von 500 bis 900 °C durchgeführt wird.

## Claims

1. A process for the preparation of fluorinated monomers by pyrolysis of fluorinated polymers in the presence of steam, which comprises carrying out the reaction in a fluidized bed reactor which contains inert, granular material as the fluidized material, and feeding in steam as the fluidizing gas.

2. The process as claimed in claim 1, wherein a polymer of tetrafluoroethylene is employed.

3. The process as claimed in claim 1 or 2, wherein the pyrolysis is carried out at a temperature of 500 to 900°C.

## Revendications

1. Procédé pour la préparation de monomères fluorés par pyrolyse de polymères fluorés en présence de la vapeur d'eau, caractérisé en ce que l'on met en oeuvre la réaction dans un réacteur à lit fluidisé, qui contient comme matière fluidisée de la matière granulée inerte, et dans lequel on introduit de la vapeur d'eau en tant que gaz de fluidisation.

2. Procédé selon la revendication 1, caractérisé en ce que l'on utilise un polymère du tétrafluoréthylène.

3. Procédé selon la revendication 1 ou 2, caractérisé en ce que l'on met en oeuvre la pyrolyse à une température de 500 à 900 °C.
